# EUROPEAN PATENT SPECIFICATION

(11) **EP 0 404 518 B1**
(45) Date of publication and mention of the grant of the patent: **08.05.1996**
(21) Application number: 90306679.3
(22) Date of filing: 19.06.1990
(51) Int. Cl.: C12Q 1/70, C12N 15/44

(54) **DNA probe for the identification of human parainfluenza type 2 virus**
DNS-Probe zum Nachweis vom Human-Parainfluenza-Typ-2-Virus
Sonde d'ADN pour la détermination du virus parainfluenza humain type 2

(30) Priority: 19.06.1989 JP 154746/89
(43) Date of publication of application: 27.12.1990
(73) Proprietor: FUJIKURA KASEI CO., LTD., Itabashi-Ku Tokyo-To (JP)
(72) Inventor: Kondo, Kunio, Mie-ken (JP); Kawano, Mitsuo, Tsu-shi, Mie-ken (JP); Ito, Yasuhiko, Tsu-shi, Mie-ken (JP)
(74) Representative: Ruffles, Graham Keith

(56) References cited:
- EP-A- 0 015 841
- GB-A- 2 161 814
- WORLD PATENTS INDEX LATEST, AN 88-210707 Derwent Publications, Ltd., London GB, week 8830
- CLINICS IN LABORATORY MEDICINE vol. 5, no. 3, September 1985, US pages 513 - 529; M.L. LANDRY et al.;
- VIRUS RESEARCH vol. 12, no. 1, 1988, AMSTERDAM NL pages 87 - 96; J.KOVAMEES ET AL.: 'Complete nucleotide sequence of the hemagglutinin-neuraminidase hn messenger rna of MUMPS virus and comparison of paramyxovirus hn proteins'
- VIROLOGY vol. 174, January 1990, NEW YORK US pages 308 - 313; M.KAWANO ET AL.: 'Sequence determination of the hemagglutinin-neuraminidase (HN) gene of human parainfluenza type 2 virus ...'
- THE JOURNAL OF GENERAL VIROLOGY vol. 71, no. 5, May 1990, READING GB pages 1163 - 1168; B.PRECIOUS ET AL: 'Sequence analysis of the HN gene of parainfluenza virus type 2'

## Description

### Background of the Invention

### Field of the Invention

This invention relates to DNA for use in test agents for the identification of human parainfluenza Type 2 virus. More particularly, it is concerned with DNA which has complementation to gene RNA of hemagglutinin neuraminidase (called HN for short hereinbelow) of human parainfluenza Type 2 virus (called PIV-2 for short hereinbelow).

Further this invention relates to a detecting method of viral infection caused by said virus.

### Description of the Prior Art

Viruses have heretofore been identified on the basis of serological properties. Methods for such identification includes those by enzyme-linked immunosorbent assay (called ELISA for short hereinbelow), neutralization reaction, complement fixation reaction, hemagglutination inhibition reaction, fluorescent antibody technique and agar precipitation reaction. All of them involve assay of the antibody to a given virus in the test material. In order to make an accurate determination the assay must be run at a stage at which antibody titer for the virus begins to increase, generally a week after infection and, in some cases, re-assay after several weeks is required. It is therefore a problem that diagnosis prior to onset of the symptoms caused by the infection or early diagnosis is hardly feasible.

For the determination of PIV-2 is usually employed the ELISA method by which antibody against PIV-2 in test specimens is assayed, for example, by adding a test specimen to a plate on which PIV-2 has been fixed, allowing a reaction to proceed, washing the plate, further adding anti-PIV 2 antibody to the plate, allowing a reaction to proceed, then adding the enzyme-labelled antibody to the reaction mixture, allowing a reaction to proceed and subsequently developing a color. This method also cannot escape from the problem as mentioned above, that is, difficulty in presymptom and early diagnoses.

The present invention aims at solving such problems and provides therefor DNA useful in test agents which makes early diagnosis of PIV-2 infections possible.

### Summary of the Invention

An object of the invention is to provide DNA according to Claim 1.

The said DNA includes the following base sequence:

10
GCACGAACCCTTAAGGTGTCGTAACGTCTCGTGACACCGGGTT

53
CAGTTCAAATATCGACCTCTAACCCAATTTAACACCCATTCTTATATAAGAAC

106
ACAGTATAATTTAATCACAAAAGACCTCAAAAACTGACACAGCTTGATCCACT

159
CAACATATAATTGTAAGATTAATAATAATGGAAGATTACAGCAATCTATCTCT

212
TAAATCAATTCCTAAAAGGACATGTAGAATCATTTTCCGAACTGCCACAATTC

265
TTGGAATATGCACATTGATTGTTCTATGTTCAAGTATTCTTCATGAGATAATT

318
CATCTTGATGTTTCCTCTGGTCTCATGGATTCCGATGATTCACAGCAAGGCAT

371
TATTC

There is further provided a method of preparing a DNA probe for human PIV-2 according to claim 2.

The DNA has complementation to gene RNA of PIV-2 HN and therefore, when used in test agents for the identification of PIV-2, direct identification of PIV-2 can be attained and the problem associated with prior-art indirect identification methods can be solved.

Another object of the invention is to provide a detecting method of viral infection caused by human prainfluenza Type 2 virus. Such a method is provided according to the method of claim 3.

### Brief Description of the Drawing

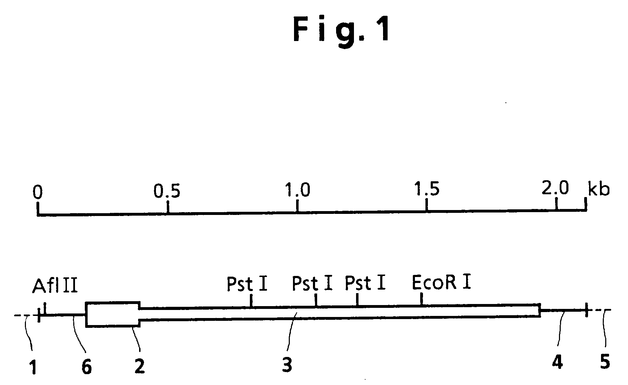

Fig. 1 is a restriction map of the cDNA region of DNA (pM 2H) containing the above-mentioned formula exhibiting complementation to PIV-2 HN gene.

### Detailed Description of the Invention

The DNA of the invention can be prepared by conventional processes without particular limitation, which include, for example,
(a) introducing cDNA prepared from mRNA of PIV-2-infected cells into an appropriate vector such as plasmid vector of Escherichia coli, cloning of the cDNA in E. coli, isolating the clone coding for PIV-2 HN gene, for example, by
   (1) screening using nucleocapsid RNA as a probe, or
   (2) screening using an oligonucleotide synthesized on the basis of the base sequence represented by the above-mentioned formula as a probe, or
   (3) screening using antibody against PIV-2,
   and separating the insert DNA from plasmid of the isolated clone,
(b) preparing the synthetic nucleotide according to the phosphamidide method (Nature, vol. 310, page 105, 1984) on the basis of the base sequence represented by the above-mentioned formula which is complementary to gene RNA of PIV-2 HN, or
(c) combining (a) and (b) above.

The DNA of the invention produced by the above-described processes can be used in the following way:

For example, the identification of PIV-2 is accomplished by the Northern hybridization (called the Northern method for short) in which the DNA obtained as above, after further cutting into fragments of ten-odd or more bases with appropriate restriction enzymes (such as PstI and EcoRI) if required, is labelled, for example, with a radioisotope for use as a test agent, the test agent is reacted with a nylon membrane or the like on which the RNA extracted from a test specimen (such as laryngeal fluid) is fixed and then judgement is made by autoradiography or other techniques.

Viral infections can be detected by the presence or absence in a test specimen of virus-constituting genome RNA or an mRNA derived from the genome RNA. The Northern hybridization is generally used for the detection of RNA of virus origin.

A probe is prepared with the DNA or a synthetic nucleotide provided by the invention and labelled, for example, with ³P. If a nucleic acid present in a test specimen undergoes hybridization with the probe, the specimen is judged as infected with PIV-2.

### [Examples]

The invention will be described in more detail in the following examples.

### (1) Preparation of RNA

Vero cells were grown in Eagle's essential medium containing 5% fetal calf serum, and the medium was then replaced by freshly prepared Eagle's essential medium containing actinomycin D (2 µg/ml), which was then inoculated with PIV-2 (Toshiba strain). Cultivation was made in the same medium for 24 hours. The virus-infected culture cells were stripped with a trypsin/EDTA mixture and then collected by centrifugation at 8000 rpm for 10 min. To the cells was added a guanidine isothiocyanate solution (6 M guanidine isothiocyanate, 5 mM sodium citrate, 0.1 M 2-mercaptoethanol and 0.5% sodium lauroylsarcosinate), and the mixture was quickly treated in a homogenizer to give a solution. The chromosome DNA was sheared by passing the solution five times through a 21-gauge needle attached to a syringe. The cytolysate thus obtained was centrifuged again at 8000 rpm. 9 ml of the supernatant was layered onto 3 ml of a 5.7 M cesium chloride solution placed in a centrifugal tube, which was ultracentrifuged in a Beckman rotor (Beckman SW40Ti rotor) at 18°C at 37000 rpm for 18 hours. After centrifugation, an RNA pellet was recovered.

From the RNA thus obtained was separated and purified polyadenylated mRNA [called poly(A+)RNA for short hereinbelow] using oligo dT cellulose Type 7 (manufactured by Pharmacia).

### (2) Preparation of cDNA library

cDNA library was synthesized according to the method of Okayama-Berg. To 4 µg of the poly(A+)RNA prepared above was added 5 µl of distilled water. The mixture was heated at 65°C for 10 min. followed by rapid cooling. Then, 10 µl of oligo dT-primed vector [oligo dT was added to KpnI site of pUC118 vector (0.8-1.2 µg/µl)], 3 µl of a solution for synthetic reaction (500 mM tris-hydrochloric acid buffer of pH 8.3, 300 mM potassium chloride, 80 mM magnesium chloride and 3 mM dithiothreitol) and 1 µl each of 20 mM dATP, 20 mM dCTP, 20 mM dGTP and 20 mM dTTP were added. Further added were 20 units of RNasin and 40 units of reverse transcriptase and then distilled water to make up the entire volume to 30 µl. The reaction was made at 42°C for 30 min to synthesize first-strand cDNA. After completion of synthesizing the first-strand cDNA, 10-30 dG bases were added using a terminal deoxynucleotidyl transferase in the presence of dGTP. Then, digestion was made with a HindIII restriction enzyme, and annealing was made with a linker having a dC base chain followed by ring closure with E. coli ligase. Finally, the second strand was synthesized with a DNA polymerase in the presence of RNase thereby preparing a perfect plasmid DNA. To 100-200 µl of competent cells (DH 1) obtained by a calcium-chloride treatment were then added 10 µl of a mixed solution of a 0.4 M magnesium chloride/0.1 M calcium chloride mixture and 0.02 µg of the DNA as prepared above, and the mixture was allowed to stand at 0°C for 40 min and then at 42°C for 90 sec followed by addition of 1.5 ml of a medium (a solution of 10 g of bactotrypton, 5 g of yeast extract and 5 g of sodium chloride in 1000 ml of distilled water). Allowing the mixture to stand at 37°C for 40 min. yielded the transformed cells.

Thus, there were obtained 1400 independent clones in the presence of ampicillin by introducing 0.02 µg of the plasmid DNA into 200 µl of the competent cells (DH 1).

### (3) Preparation of nucleocapsid RNA probe

To PIV-2 infected vero cells was added a solution (0.15 M sodium chloride, 0.05 M tris-hydrochloric acid buffer) containing 0.6% Nonidet P-40 and 10 mM vanadium ribonucleotide complex. The mixture was solubilized by pipetting in ice for one hour and centrifuged at 8000 rpm for 10 min. Onto 1 ml of a 40%, 2.5 ml of a 30% and 1 ml of a 25% aqueous solutions of cesium chloride layered in this order in a centrifugal tube was further layered 9 ml of the supernatant obtained above by centrifugation, and the layered solutions were subjected to equilibrium density-gradient centrifugation in a Beckman™ rotor SW40Ti at 37000 rpm for 18 hours at 16°C. There was recovered a virus nucleocapsid band formed in the layer of 30% aqueous cesium chloride solution, which was then subjected to proteolysis with 0.1% SDS and proteinase K (2.5 mg/ml) at 56°C for 15 min. Subsequent treatments with phenol and a phenol/chloroform mixture produced a nucleocapsid RNA. To the nucleocapsid RNA thus produced was added a 50 mM tris-hydrochloric acid buffer of pH 9.7, and the mixture was heated at 95°C for 10 min. followed by cooling slowly to room temperature. To 20 µl of the RNA solution were added 10 µl of a buffer (250 mM tris-hydrochloric acid, 50 mM magnesium chloride, 25 mM DTT, 7.5 mM spermine and 500 mM potassium chloride), 3 µl of ³P ATP (100 µCi/600 pM) and 2 units of T₄-DNA kinase, and the mixture was diluted with distilled water to a total volume of 50 µl. The resulting mass was allowed to react at 37°C for one hour to give a nucleocapsid RNA probe.

### (4) Colony hybridization

100-200 µl of the transformed cells prepared above were seeded onto agar plates each 15 cm in diameter (10 g of bactotrypton, 5 g of yeast extract, 5 g of sodium chloride and 15 g of agar added and diluted with distilled water to a total volume of 1000 ml) containing ampicillin (120 µg/ml) and incubated at 37°C for 12 hours. The formed colonies were transferred to nitrocellulose membranes, which were incubated at 37°C for 6 hours on fresh agar plate. The membranes were then treated with a 0.5 N sodium hydroxide/1.5 M sodium chloride mixture for 10 min and further with a 0.5 M tris-hydrochloric acid buffer of pH 8.0 for 10 min and subsequently rinsed with a mixed solution containing a 0.3 M sodium chloride/0.03 M sodium citrate for 5 min. After rinsing, the membranes were air dried and suction baked at 80°C for one hour to fix the DNA on the membranes.

To the membrane thus prepared was added a prehybridization solution composed of 1 ml of denatured salmon RNA (1 mg/ml), 7.5 ml of SSPE solution (3.6 M sodium chloride, 200 mM sodium dihydrogen phosphate and 20 mM EDTA), 1.25 ml of Denhardt's solution (2% BSA, 2% Ficoll and 2% polyvinylpyrrolidone) and 1.25 ml of 10% SDS per 5 membranes followed by reaction at 42°C for 12 hours. Then, the nucleocapsid RNA probe mentioned above was added to a fresh prehybridization solution prepared as above at a level of 5,000,000 Ci/ml followed by reaction at 42°C for 18 hours. After completion of the reaction, the membranes were washed twice in a 1:20-diluted SSPE solution containing 0.1% SDS at 42°C for 5 min and then twice in a 1:200-diluted SSPE solution containing 0.1% SDS at 42°C for 15 min. After completion of the washing, the membrane was air dried and subjected to autoradiography using X-ray film (RX5, manufactured by Kodak) at -80°C for 12 hours. There were obtained several positive clones.

### (5) Northern method

The northern method was employed for the positive clones obtained above. Electrophoresis was conducted in 1.5% agarose gel respectively of the PIV-2-infected cell-derived poly(A+)RNA produced by the procedures under (1) above, a virus-noninfected cell-derived poly(A+)RNA and an rRNA marker. After electrophoresis was completed, the RNA was transferred from agarose gel to a nylon membrane (Hybond-N, manufactured by Amersham, Inc.), which was air dried and UV irradiated to produce a covalent bond of the RNA with the nylon membrane. Hybridization was then carried out using the above-obtained positive clones respectively as a probe.

Thus, the nylon membrane was allowed to react in the above-mentioned prehybridization solution at 42°C for 12 hours. To the prehybridization solution was added a ³P-labelled probe prepared from each of the positive clones at a level of 1,000,000 Ci/ml followed by reaction at 42°C for 18 hours. After completion of the reaction, washings were made in the same way as under (4) above, and autoradiography was made using X-ray film. There was then obtained one clone that hybridizes at a site corresponding to the base 2100, which was named pM2H.

It is noted that the probe for each of the positive clones was obtained by digesting each clone with HindIII and EcoRI, then separating inserted DNA by electrophoresis using low melting agarose, extracting it and subjecting it to random prime labelling using ³P dCTP.

### (6) Preparation of a restriction map of the DNA exhibiting complementation to PIV-2 HN gene and determination of the base sequence

The clone pM2H obtained above under (5) was cleaved with various restriction enzymes to prepare its restriction map. The results are shown in Fig. 1. In the figure, 1 and 5 indicate the vector DNA, 4 and 6 the non-coding region and 2 and 3 the coding region. Among them, 2 and 6 represent the DNA fragment exhibiting complementation to PIV-2 HN gene RNA.

Fragments produced by cleaving the clone pM2H with each of the restriction enzymes as shown in Fig. 1 were respectively introduced into multicloning sites of the plasmid pUC118 and sequenced using Sequence™ kit (manufactued by TOYOBO CO., LTD.). Deletion mutants were also prepared and sequenced using deletion kit for kilo sequencing (manufactured by Takara Shuzo Co., Ltd.) to determine the base sequence.

The results are as shown by the above-mentioned formula.

### (7) Application to the test agent (identification of PIV-2)

A probe was prepared by cleaving the clone pM2H obtained above under (4) followed by electrophoresis using low melting agarose to separate a portion of the inserted DNA, extracting it and subjecting it to random prime labelling using ³P. Then, 0.35 µg each of the PIV-2-infected cell-dirived RNA obtained by the procedures under (1) above and the virus-noninfected cell-derived RNA was blotted on a nylon membrane, which was air dried, UV irradiated and subjected to reactions and radioautography in exactly the same way as in the Northern method described above under (5). From obtained autoradiographic image, there was observed positive reaction in the area blotted with the PIV-2-infected cell-derived RNA thereby demonstrating the infection with PIV-2. On the contrary, there was observed no reaction at all in the area blotted with the virus-noninfected cell-derived RNA.

### [Effect of the invention]

The DNA fraction of the above-mentioned formula provided by the present invention exhibits complementation specifically to PIV-2 HN gene and is very valuable for use in test agents for early diagnosis of PIV-2 infections.

It will of course be recognised that a DNA sequence of some 2000 base pairs may contain sections which are not essential for hybridization with RNA from PIV-2 infected cells and, in practice, a fragment derived from restriction enzyme digest of the DNA sequence will suffice.

Analogues with a DNA sequence sufficiently close for hybridization with the test RNA are also part of the scope of this invention.

## Claims

1. DNA for use in a test agent for the identification of human parainfluenza type 2 virus (PIV-2), said DNA corresponding to regions 2 and 6 of the following restriction map of a cloned cDNA containing the PIV-2 hemagglutinin neuraminidase gene: 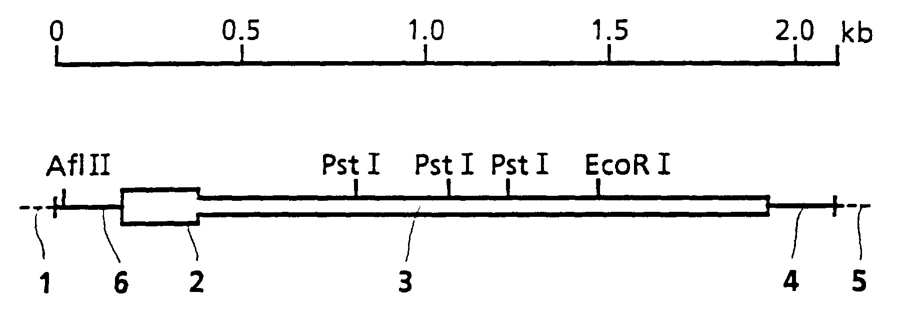 wherein 1 and 5 indicate vector DNA, 4 and 6 the non-coding region and 2 and 3 the coding region, said regions 2 and 6 including the following base sequence: 
10
GCACGAACCCTTAAGGTGTCGTAACGTCTCGTGACACCGGGTT

53
CAGTTCAAATATCGACCTCTAACCCAATTTAACACCCATTCTTATATAAGAAC

106
ACAGTATAATTTAATCACAAAAGACCTCAAAAACTGACACAGCTTGATCCACT

159
CAACATATAATTGTAAGATTAATAATAATGGAAGATTACAGCAATCTATCTCT

212
TAAATCAATTCCTAAAAGGACATGTAGAATCATTTTCCGAACTGCCACAATTC

265
TTGGAATATGCACATTGATTGTTCTATGTTCAAGTATTCTTCATGAGATAATT

318
CATCTTGATGTTTCCTCTGGTCTCATGGATTCCGATGATTCACAGCAAGGCAT

371
TATTC
 or an analogue containing a fragment thereof capable of hybridizing with RNA from cells infected with human parainfluenza type 2 virus.

2. A method of preparing a DNA probe according to Claim 1 for human parainfluenza Type 2 virus (PIV-2) comprising the steps of
(1) obtaining polyadenylated mRNA (poly(A+)RNA) from PIV-2 infected cells;
(2) preparing cDNA from the poly(A+)RNA obtained and transforming competent cells therewith;
(3) preparing a nucleocapsid RNA probe from PIV-2 infected cells;
(4) hybridizing the transformed cells from step (2) with the probe of step (3) to obtain positive clones;
(5) hybridizing the poly(A+)RNA using a positive clone as probe to identify a clone hybridizing to said poly(A+) RNA; and
(6) labelling said probe from step (5), or a fragment thereof, with a radiosotope.

3. A method of detecting viral infection caused by human parainfluenza Type 2 virus comprising reacting a probe obtained by a method of claim 2, with RNA extracted from a test specimen and examining the result by autoradiography.

4. A method of detecting viral infection caused by human parainfluenza Type 2 virus which comprises labelling the DNA or analogue thereof according to Claim 1 with a radiosotope to obtain a test agent, reacting the test agent with RNA extracted from a test specimen and making an autoradiographic assessment on the reaction.

## Patentansprüche

1. DNS zur Verwendung in einem Prüfmittel zum Nachweis vom Human-Parainfluenza-Typ-2-Virus (PIV-2), wobei die DNS den Regionen 2 und 6 der folgenden Restriktionskarte einer geklonten cDNS entspricht. die das PIV-2-Hämagglutinin-Neuraminidase-Gen enthält: 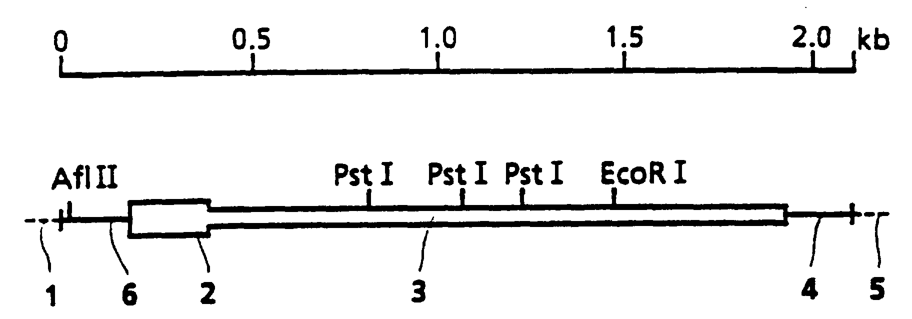 wobei 1 und 5 den DNS-Klonierungsvektor anzeigen, 4 und 6 die nichtcodierende Region und 2 und 3 die codierende Region, wobei die Regionen 2 und 6 die folgende Basensequenz: 
10
GCACGAACCCTTAAGGTGTCGTAACGTCTCGTGACACCGGGTT

53
CAGTTCAAATATCGACCTCTAACCCAATTTAACACCCATTCTTATATAAGAAC

106
ACAGTATAATTTAATCACAAAAGACCTCAAAAACTGACACAGCTTGATCCACT

159
CAACATATAATTGTAAGATTAATAATAATGGAAGATTACAGCAATCTATCTCT

212
TAAATCAATTCCTAAAAGGACATGTAGAATCATTTTCCGAACTGCCACAATTC

265
TTGGAATATGCACATTGATTGTTCTATGTTCAAGTATTCTTCATGAGATAATT

318
CATCTTGATGTTTCCTCTGGTCTCATGGATTCCGATGATTCACAGCAAGGCAT

371
TATTC
 oder ein Analogon. das ein Fragment davon enthält. das zur Hybridisierung mit der RNS aus den mit dem Human-Parainfluenza-Typ-2-Virus infizierten Zellen fähig ist, einschließen.

2. Verfahren zur Herstellung einer DNS-Probe nach Anspruch 1 für den Human-Parainfluenza-Typ-2-Virus (PIV-2), umfassend die Schritte der
(1) Gewinnung von polyadenylierter mRNS (Poly(A+)RNS) aus PIV-2-infizierten Zellen;
(2) Herstellung von cDNS aus der gewonnenen Poly(A+)RNS und Umwandlung kompetenter Zellen damit;
(3) Herstellung einer Nukleokapsid-RNS-Probe aus PIV-2-infizierten Zellen;
(4) Hybridisierung der umgewandelten Zellen aus Schritt (2) mit der Probe von Schritt (3) zur Gewinnung positiver Klone;
(5) Hybridisierung der Poly(A+)RNS unter Verwendung eines positiven Klons als Probe zur Identifizierung eines Klons unter Hybridisierung zu der Poly(A+)RNS; und
(6) Markierung der Probe aus Schritt (5), oder eines Fragments davon, mit einem Radioisotop.

3. Verfahren zum Nachweis einer durch den Human-Parainfluenza-Typ-2-Virus verursachten Virusinfektion, umfassend die Umsetzung einer Probe, gewonnen durch ein Verfahren nach Anspruch 2, mit der aus einer Versuchsprobe extrahierten RNS und Untersuchung des Ergebnisses durch Autoradiographie.

4. Verfahren zum Nachweis einer durch den Human-Parainfluenza-Typ-2-Virus verursachten Virusinfektion, das die Markierung der DNS oder eines Analogons davon nach Anspruch 1 mit einem Radioisotop zur Gewinnung eines Prüfmittels. Umsetzung des Prüfmittels mit aus einer Versuchsprobe extrahierter RNS und Erstellung einer autoradiographischen Beurteilung der Umsetzung umfaßt.

## Revendications

1. ADN pour l'utilisation dans un agent test pour l'identification du virus parainfluenza humain de type 2 (PIV-2), ledit ADN correspondant aux régions 2 et 6 de la carte de restriction suivante d'un ADNc cloné contenant le gène de l'hémagglutinine neuramidinase du PIV-2 : 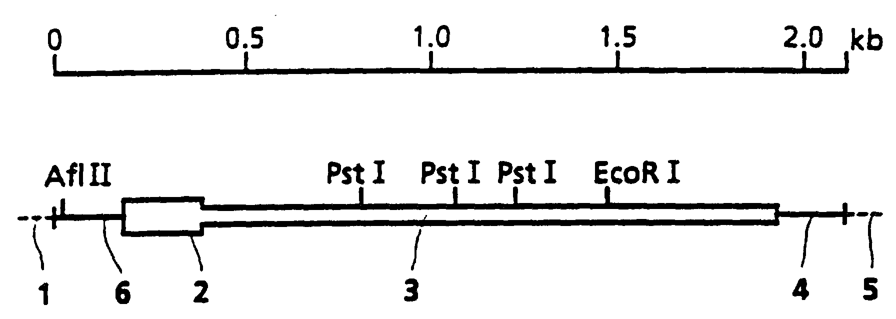 dans laquelle 1 et 5 indiquent l'ADN vecteur, 4 et 6 la région non codante et 2 et 3 la région codante, lesdites régions 2 et 6 incluant la séquence de bases suivante: 
10
GCACGAACCCTTAAGGTGTCGTAACGTCTCGTGACACCGGGTT

53
CAGTTCAAATATCGACCTCTAACCCAATTTAACACCCATTCTTATATAAGAAC

106
ACAGTATAATTTAATCACAAAAGACCTCAAAAACTGACACAGCTTGATCCACT

159
CAACATATAATTGTAAGATTAATAATAATGGAAGATTACAGCAATCTATCTCT

212
TAAATCAATTCCTAAAAGGACATGTAGAATCATTTTCCGAACTGCCACAATTC

265
TTGGAATATGCACATTGATTGTTCTATGTTCAAGTATTCTTCATGAGATAATT

318
CATCTTGATGTTTCCTCTGGTCTCATGGATTCCGATGATTCACAGCAAGGCAT

371
TATTC
 ou un analogue contenant un fragment de celui-ci, capable de s'hybrider avec l'ARN provenant de cellules infectées avec le virus parainfluenza humain de type 2.

2. Procédé de préparation d'une sonde d'ADN selon la revendication 1 pour le virus parainfluenza humain de type 2 (PIV-2), comprenant les étapes
(1) d'obtention d'ARNm polyadénylé (poly(A+)ARN) à partir de cellules infectées par le PIV-2 ;
(2) de préparation d'ADNc à partir du poly(A+)ARN obtenu et de transformation de cellules compétentes avec celui-ci ;
(3) de préparation d'une sonde d'ARN de nucléocapside à partir de cellules infectées par le PIV-2 ;
(4) d'hybridation des cellules transformées de l'étape (2) avec la sonde de l'étape (3) pour obtenir des clones positifs ;
(5) d'hybridation du poly(A+)ARN en utilisant un clone positif comme sonde pour identifier un clone s'hybridant audit poly(A+)ARN ; et
(6) de marquage de ladite sonde de l'étape (5), ou d'un fragment de celle-ci, avec un radio-isotope.

3. Procédé de détection d'une infection virale provoquée par le virus parainfluenza humain de type 2, comprenant la réaction d'une sonde obtenue par un procédé de la revendication 2 avec de l'ARN extrait d'un spécimen test, et l'examen du résultat par autoradiographie.

4. Procédé de détection d'une infection virale provoquée par le virus parainfluenza humain de type 2, qui comprend le marquage de l'ADN ou d'un analogue de celui-ci selon la revendication 1 avec un radio-isotope pour obtenir un agent test, la réaction de l'agent test avec de l'ARN extrait d'un spécimen test, et la réalisation d'une évaluation autoradiographique de la réaction.
